(19) Europäisches Patentamt
European Patent Office
Office européen des brevets

(11) EP 0 502 510 B2

(12) NEW EUROPEAN PATENT SPECIFICATION

(45) Date of publication and mention of the opposition decision:
**14.01.2004 Bulletin 2004/03**

(45) Mention of the grant of the patent:
**20.12.1995 Bulletin 1995/51**

(21) Application number: **92103702.4**

(22) Date of filing: **04.03.1992**

(51) Int Cl.[7]: **B01J 23/78**, C07C 5/333, C07C 15/46

# (54) Alkyl aromatic hydrocarbon dehydrogenation catalyst and production method thereof

Katalysator zur Dehydrierung eines alkylierten aromatischen Kohlenwasserstoffes und Verfahren zur Herstellung desselben

Catalyseur de déshydrogénation d'hydrocarbures alkyl- aromatiques et procédé de préparation dudit catalyseur

(84) Designated Contracting States:
**DE FR GB IT**

(30) Priority: **05.03.1991 JP 12318591**

(43) Date of publication of application:
**09.09.1992 Bulletin 1992/37**

(73) Proprietor: **Süd-Chemie Catalysts Japan Inc.**
**Tokyo (JP)**

(72) Inventors:
- **Murakami, Akira**
  **Nei-gun, Toyama-ken (JP)**
- **Unei, Hidemi**
  **Higashimurayama-shi, Tokyo (JP)**
- **Teranishi, Masayuki**
  **Kasukabe-shi, Saitama-ken (JP)**
- **Ohta, Masaki**
  **Nei-gun, Toyama-ken (JP)**

(74) Representative:
**Splanemann Reitzner Baronetzky Westendorp**
**Patentanwälte**
**Rumfordstrasse 7**
**80469 München (DE)**

(56) References cited:
**CS-A- 168 220**
**DE-B- 1 181 694**
**GB-A- 680 509**
**US-A- 3 437 703**
**US-A- 3 904 552**

- **PATENT ABSTRACTS OF JAPAN, vol. 13, no. 158 (C-586)[3506], 1988;& JP-A-63 316 746 (NIPPON STEEL CHEM. CO.) 26-12-1988**
- **United Catalyst Inc., G-64 and G-84 Product Bulletin**

EP 0 502 510 B2

Printed by Jouve, 75001 PARIS (FR)

## Description

The present invention relates to a catalyst for producing alkenyl aromatic hydrocarbons such as styrene and the like by means of dehydrogenation of alkyl aromatic hydrocarbons such as ethylbenzene and the like, in the presence of steam.

Styrene is usually produced by dehydrogenation of ethylbenzene, which is used as a raw material monomer for synthetic rubber, ABS resins, polystyrene and the like, so that its production amount is increasing year by year.

The ethylbenzene dehydrogenation reaction is an endothermic reaction accompanied by expansion of volume as shown by the following reaction formula:

$$C_6H_5 \cdot C_2H_5 \rightarrow C_6H_5 \cdot C_2H_3 + H_2 \text{ - 113 kilojoules/mol}$$

This reaction was actively studied in 1940th in the United States in order to meet the requirements for the production of synthetic rubber, during which there has been technically established a system in which ethylbenzene is catalytically dehydrogenated under steam dilution as industrially carried out at present, resulting in occupying a position as a representative production method of styrene.

The volume is expanded in this reaction, so that it is advantageous from the viewpoint of chemical equilibrium to dilute the reactants with steam, and the steam dilution has the following advantages other than the above.

(a) The reaction is performed at a high temperature of 550 °C to 650 °C, so that steam can be utilized as a heat source for heating ethylbenzene.

(b) Although carbonaceous substances deposit on the catalyst due to side reactions, a water gas reaction with steam can be utilized for removing them, whereby continuous use can be conducted without regeneration of the catalyst.

(c) The steam as the diluent can be easily separated from the product only by liquefying the product.

As described above, the dehydrogenation reaction system in the presence of steam is an industrially excellent production method in which styrene can be continuously produced under advantageous conditions from the viewpoint of chemical equilibrium, and the foregoing operation method has become technically available owing to the fact that it has been revealed that the iron oxide-potassium oxide system catalyst stably maintains its high performance without being poisoned by steam; however, before the catalyst became industrially available, many further improvements in performance had been contemplated, during which addition of various promoter components had been investigated.

The role of each catalyst component has been scholarly elucidated under a situation of reaction, wherein the component which has an activity on the dehydrogenation reaction itself is partially reduced iron oxide, and potassium oxide acts as the promoter to enhance the activity of iron oxide and promotes the water gas reaction of steam with the carbonaceous substances deposited on the surface of the catalyst so as to prevent time-dependent deterioration of the activity, and other promoter components are added in order that the activity and selectivity or the thermal stability, mechanical strength stability and the like of the catalyst are increased.

The catalyst is usually produced such that iron oxide or an iron compound as its precursor, a potassium compound and other promoter component oxides or precursor compounds thereof are mixed and kneaded in the presence of moisture, and then extrusion molding, drying and calcination are performed.

Those used as raw iron materials are red iron oxide (hematite) or yellow iron-oxide hydroxide (goethite) as its precursor compound and the like, and the raw potassium materials are those which can be decomposed into potassium oxide by calcination, for which any compound can be used provided that no component which gives a poisoning action is allowed to remain in the catalyst. However, potassium hydroxide, potassium carbonate or the like is usually used.

Iron oxide and potassium oxide are essential components provided that the ethylbenzene dehydrogenation reaction is performed in the presence of dilution steam, and the combination of the both components greatly enhances the activity of iron oxide as compared with the case in which it is used alone, however, only the both components were insufficient for use as an industrial catalyst, and in order to improve the activity as well as the selectivity, stability of catalyst structure, mechanical strength and the like, various promoter components to meet with the object have been added and supplied as commercial catalysts.

As the promotor components to be added, for example Ce, Cr and the like are known as components for increasing the activity, Ca, V, Mo, W and the like as components for increasing the selectivity. As the prior art in which these elements are used, there is proposed the addition of Ce, Mo, Ca, Mg, or Cr in US-A-5 023 225, the addition of Cr, Mo, W, and Al in DE-C-40 25 931, and the addition of Ca, Ce, Ge, Sn, Pb and the like in JP-A-64-27646 respectively. As components for contributing to the structural stability of the catalyst are known Cr, Mg, and the like which are disclosed in US-A-5 023 225 or DE-A-4 025 931 together with the components for increasing the performance. As a component for stablilizing the catalyst structure being different from these elements, addition of Ti is disclosed in CS-A-168 220 and 174 488 respectively.

The addition of these promoter components greatly increases the catalyst performance and improves the stability of the catalyst structure or mechanical strength. However, the dehydrogenation catalysts have a high alkali content and are used at a high reaction temperature in spite of the high alkali content, so that problems such as migration of the alkali metal in the catalyst layer and the like are likely to take place in practical operation, which results in the decrease in catalyst performance or the increase in pressure drop due to blockade of the catalyst layer. Hence, the danger of giving trouble for the operation of the equipment is included, while its activity is fairly low as compared with the equilibrium conversion ratio of ethylbenzene at a practical industrial reaction temperature. Thus, there remains room for improvement from a viewpoint of performance.

When the ethylbenzene dehydrogenation reaction is considered from an industrial viewpoint, if the activity can be increased without deteriorating the selectivity of the catalyst, then not only the yield of styrene can be increased, but also an operation under more moderate condition is possible, so that it becomes possible to provide countermeasures for reducing various operational problems concerning the catalyst, such as the decrease in activity due to sintering of iron oxide on account of thermal influences or migration of alkali metal, the increase in pressure drop due to scattering of alkali metal and the like.

The present inventors have considered the fact that the existing catalysts have room for improving performances, taken notice of increasing the activity of the ethylbenzene dehydrogenation catalyst without sacrificing the selectivity, and have investigated from various viewpoints mainly for addition of another. component to the iron oxide-potassium oxide system catalyst.

Now it has been found that when a small amount of titanium oxide is added together with other promoter components, the performance remarkably increases, and further its effect not only increases the activity, but also fairly increases the selectivity at the same conversion ratio. It was also found that its effect does not depend on the method of how the titanium is added. Accordingly, the invention provides a catalyst as defined in claim 1.

Titanium oxide, when added in a small amount 0.15 to 0.95 wt.% provides an unexpected effect of increasing the performance, the maximum performance being exhibited by a specific amount that provides a typical promoter effect on the increase in performance. This particular result could not be expected in view of CS-A-168220 and 174488 in which 1 to 10 wt.-% of titanium oxide is aded to a catalyst consisting of iron oxide, potassium oxide and vanadium oxide in order to provide the structural stability of the catalyst.

Preferred embodiements of the present invention are recited in the dependent claims.

The present inventors have investigated catalysts containing Ce, Mo, alkaline earth metals and the like as the promoter components which are considered to give a high activity and high selectivity in order to further increase the activity. It has been found that when various catalyst raw materials including iron oxide are subjected to wet mixing and kreading followed by extrusion molding and subsequent drying and calcination so as to produce a catalyst, the addition and mixing and kneading of titanium oxide can remarkably improve its performance. Therefore another object of the invention is a method as recited in claim 5.

In such methods titanium oxide itself or a precursor of titanium oxide is added during the wet mixing and kreading step of each of the catalyst materials, or the titanium oxide is added beforehand to iron oxide by initially adding and dissolving water-soluble titanium salts in an aqueous iron salt solution and then obtain iron oxide by precipitation or heat decomposition which are ordinary industrial methods for producing iron oxide. However, even industrial iron oxide can be used as the iron oxide, provided that it already contains titanium oxide in an amount suitable for the present object.

As the iron salts for producing iron oxide, any salt can be used provided that it is water-soluble, such as iron sulfate, iron nitrate, iron halides and the like. However, when iron oxide is produced by precipitation, iron sulfate is usually used for economic reasons. The iron oxide is produced by a neutralization reaction with a basic substance, wherein it is necessary to thoroughly wash the precipitate, in order to keep the remaining sulfur in the iron oxide as low as possible. When iron oxide is produced by heat decomposition, iron chloride is usually used for economic reasons and easiness of heat decomposition. However, in order to keep the remaining chloride in the iron oxide as low as possible, the heat decomposition should be carried out to a sufficient extent.

The iron raw material to be used for the present catalyst is usually iron oxide, however, iron oxide precursors which can be converted into oxide during the calcination of the catalyst, namely iron compounds such as goethite or basic iron carbonate and the like, or mixtures of these iron compounds and iron oxide can also be used.

The catalyst is finally calcined, so that the components added as the promoter components are not necessarily oxides, and any compound can be used provided that it can be decomposed into an oxide by heat treatment. However, it is necessary not to use a component which acts as a catalyst poison, and hence from a viewpoint of availability, economy or the like, it is usually preferable to use hydroxides or carbonates as starting materials for the potassium, cerium and alkaline earth metal components and to use ammonium paramolybdate or molybdenum oxide for the molybdenum component.

The ranges of each of the catalyst components are defined in claim 1. For example, they may be (calculated as oxides), as follows:

| | |
|---|---|
| $Fe_2O_3$ | 40 to 90 wt.% |
| $K_2O$ | 5 to 30 wt.% |
| $Ce_2O_3$ | 2 to 20 wt.% |
| $MoO_3$ | 1 to 10 wt.% |
| Alkaline earth metal oxide | 1 to 10 wt.% |

The amount of titanium oxide added as another component is in the range of 0.005 to 0.95 wt. % (with all components calculated as oxides) irrevant of the adding method or form of titanium compound to be added.

If the amount of titanium oxide added is not more than 0.15 wt.%, the increase in performance owing to its addition is not sufficient, while if the amount thereof is more than 0.95 wt.%, the selectivity is greatly improved but the effect of increasing the activity is lowered, and further the long-term stability is lost which is a serious problem from a practical viewpoint, resulting in a catalyst having a considerable time-dependent decrease in activity so that no catalyst can be obtained which can be available for practical use.

When titanium is added during the mixing and kneading of the catalyst materials, titanium raw materials such as titanium oxide or titanium compounds decomposable to titanium oxide at the final calcination step may be used, which should not contain components which act as a catalyst poison. For example, titanium nitrate, titanium hydroxide, various titanium alkoxides and the like can be used. When titanium is initially added to iron oxide, i.e. in the case of iron oxide produced by precipitation, the precipitate containing titanium can be subjected to removal of impurities by washing with water, so that any titanium compound can be used provided that it is water-soluble. For example, titanium compounds such as titanium sulfate, titanium halides, titanium nitrate and the like can be used. On the other hand, if the iron oxide is produces by thermal decomposition, any titanium compound can be used provided that it is water-soluble. However, it is preferable to use a titanium halide because of its ease of thermal decomposition.

The catalyst raw materials including iron oxide are subjected to wet mixing and kneading, wherein the amount of water to be added during mixing and kneading should be appropriate for the extrusion molding in the following step. The amount of water is different depending on the types of materials to be used; however, it is usually added in a range of 10 to 30 wt.%.

Drying is sufficient if the free water contained in the extrusion molding product can be removed, which is usually performed at a temperature of 80 to 200 °C, preferably 100 to 150 °C. Calcination is carried such that each of the catalyst precursors contained in the dry materials is thermally decomposed and the mechanical strength of the catalyst of the catalyst pellets is improved as well as the performance thereof is increased. Calcination is usually performed in a range of 400 to 1000 °C, preferably 500 to 900 °C.

The calcination temperature is important for obtaining a catalyst having a good quality, and hence a temperature of not more than 400 °C is insufficient for converting each of the catalyst component precursors into oxides, while a temperature of more than 1000 °C promotes the crystal growth of iron oxide resulting in the decrease of activity, which is not preferable.

When measuring the performance and the performance change with time on stream by conducting the ethylbenzene dehydrogenation reaction using a reaction apparatus of the atmospheric flow system, it has been found that the effect of addition of a small amount of titanium oxide on the increase in performance is remarkable.

There is an increase of the selectivity at the same conversion ratio and a remarkable increase of the activity, the performance being stable over an extended period of time.

As described above, it had been found that the addition of a small amount of titanium oxide remarkably improves the performance of the catalyst which uses cerium, molybdenum, an alkaline earth metal as promoting agents. The present inventors further investigated chromium containing catalysts in order to improve their performance further.

The chromium containing catalysts can be also basically produced by the wet mixing and kneading of each of the catalyst component oxides or precursor compounds thereof followed by extrusion molding and subsequent drying and calcination, so that the materials which can be used for the catalysts containing cerium, molybdenum and alkaline earth metal can be used, while as raw materials for the chromium component there are used various chromate salts, especially alkali, ammonium and other chromates or dichromates. As other chromium compounds, there can be used oxides of chromium, such as chromium oxide, chromic anhydride and the like.

Thus, the present inventors prepared chromium containing catalysts in which titanium oxide is added by the same treatment methods as used for the catalysts containing cerium, molybdenum and alkaline earth metal.

Using the resulting catalysts, the ethylbenzene dehydrogenation reaction was carried out with a reaction apparatus of the atmospheric flow system, and the performance was evaluated, showing that the addition of a small amount of titanium oxide remarkably improves the performance of the chromium containing catalyst in the same manner as in the case of the catalyst containing cerium, molybdenum, and alkaline earth metal. It has been confirmed that the effect thereof is not only to increase the activity but also to increase the selectivity at the same conversion ratio, and the performance is stable for a long period.

Incidentally, the catalyst according to the present invention is not limited to the utilization as a catalyst for producing styrene by dehydrogenation of ethylbenzene only, but it can also be used for the production od various alkenyl aromatic compounds produced by similar dehydrogenation reaction systems, namely divinylbenzene from diethylbenzene, a-methylstyrene from cumene and the like.

According to the present invention, a small amount of titanium oxide is added to the catalyst consisting of iron oxide, potassium oxide and various promoter components, whereby the catalyst activity can be greatly increased, as well as the selectivity at the same conversion ratio can be increased, so that the yield of styrene can be increased without changing operation conditions. Occasionally it becomes possible to operate under more moderate conditions, and hence operation problems such as the decrease in activity due to the crystal growth of iron oxide because of thermal influences, the increase in pressure drop resulting form diffusion of potassium and the like can be reduced.

Next, the present invention will be illustrated in accordance with the Examples, wherein the performance evaluation was carried out under the following conditions:

Performance evaluation conditions:

| | |
|---|---|
| Catalyst volume (cc) | 100 |
| $H_2O$/ethylbenzene (weight ratio) | 2.0 |
| Reaction temperature (°C) | 570, 600, 620 |
| Reaction time (Hr.) | 100 |

the conversion (%) and the selectivity (%) for representing performances are calculated with the following equations, respectively,

$$\text{Conversion (\%)} = [(A-B)/A] \times 100$$

$$\text{Selectivity (\%)} = [C/(A-B)] \times 100$$

the performance change with time on stream was measured at a reaction temperature of 620 °C.

Here, A, B and C represent the following substance concentrations, respectively.

A: ethylbenzene concentration at the catalyst layer inlet (wt.%)
B: ethylbenzene concentration at the catalyst layer outlet (wt.%)
C: styrene concentration at the catalyst layer outlet (wt.%)

Example 1 (Not according to the claims)

500 g of red iron oxide (hematite crystal structure), 252 g of potassium carbonate, 25 g of magnesium carbonate, 55.2 g of cerium hydroxide, 21 g of molybdenum oxide and 0.7 g of titanium oxide were weighed and introduced into a kneader. To this mixture there was gradually added pure water with mixing to give a paste which was then subjected to extrusion molding to a 1/8 inch (3.2 mm) size, dried in a dryer at 100 to 120 ° C overnight, and thereafter transferrred to an electric furnace, and calcined at 600 °C for 4 hours.

The obtained catalyst had the following composition:

Catalyst composition

| | |
|---|---|
| $Fe_2O_3$ | 67.01 wt.% |
| $K_2O$ | 23.14 |

(continued)

| | |
|---|---|
| $Ce_2O_3$ | 5.36 |
| $MoO_3$ | 2.81 |
| MgO | 1.59 |
| $TiO_2$ | 0.094 |

The performance evaluation results are shown in Table 1.

Examples 2-3 Catalysts of Examples 2 and 3 were prepared by exactly the same treating procedure as that of Example 1 except that, 3.0 g or 6.5 g of titanium oxide were added during the wet mixing and kneading of the catalyst materials including iron oxide in Example 1.

The obtained catalysts had the following compositions:

Catalyst composition

| Component (wt.%) | Example 2 | Example 3 |
|---|---|---|
| $Fe_2O_3$ | 67.47 | 66.82 |
| $K_2O$ | 22.53 | 22.93 |
| $Ce_2O_3$ | 5.28 | 5.18 |
| $MoO_3$ | 2.67 | 2.64 |
| MgO | 1.63 | 1.55 |
| $TiO_2$ | 0.42 | 0.88 |

The performance evaluation results are shown in Table 1, and the measurement results of the performance change with time on stream for Example 2 are shown in Table 2.

Comparative example 1

The catalyst of Comparative example 1 was prepared by exactly the same treating procedure as that of Example 1 except that no titanium oxide was added during the wet mixing and kneading of the catalyst materials including iron oxide in Example 1.

The obtained catalyst had the following composition:

Catalyst composition

| | |
|---|---|
| $Fe_2O_3$ | 67.43 wt.% |
| $K_2O$ | 22.87 |
| $Ce_2O_3$ | 5.42 |
| $MoO_3$ | 2.58 |
| MgO | 1.70 |

The performance evaluation results are shown in Table 1.

Comparative example 2

The catalyst of Comparative example 2 was prepared by exactly the same treating procedure as that of Example 1 except that 23.0g of titanium oxide were added during the wet mixing and kneading of the catalyst materials including iron oxide in Example 1.

The obtained catalyst had the following composition:

Catalyst composition

| | |
|---|---|
| $Fe_2O_3$ | 65.62 wt.% |
| $K_2O$ | 22.06 |
| $Ce_2O_3$ | 5.09 |
| $MoO_3$ | 2.66 |
| MgO | 1.54 |
| $TiO_2$ | 3.03 |

The performance evaluation results are shown in Table 1, and the measurement results of the performance change with time on stream are shown in Table 2.

Example 4 (Not according to the claims)

The catalyst of Example 4 was prepared by exactly the same treating procedure as that of Example 1 except that 1.57 g of tetrabutoxy titanate [$(n-C_4H_9O)_4Ti$] was used during the wet'mixing and kneading of the catalyst materials including iron oxide in Example 1.

The obtained catalyst had the following composition:

Catalyst composition

| | |
|---|---|
| $Fe_2O_3$ | 67.57 wt.% |
| $K_2O$ | 23.02 |
| $Ce_2O_3$ | 5.08 |
| $MoO_3$ | 2.57 |
| MgO | 1.71 |
| $TiO_2$ | 0.048 |

The performance evaluation results are shown in Table 1.

Example 5 (Not according to the claims)

500 g of red iron oxide (hematite crystal structure), 84 g of potassium carbonate, 24.0 g of ammonium dichromate and 0.5 g of titanium oxide were weighed and introduced into a kneader. To this mixture there was gradually added pure water with mixing to give paste which was then subjected to extrusion molding to a 1/8 inch (3.2) size with an extruder, dried in a dryer at 100 to 120 °C overnight, and thereafter transferred to an electric furnace, and calcined at 600 °C for 4 hours.

The obtained catalyst had the following composition:

Catalyst composition

| | |
|---|---|
| $Fe_2O_3$ | 87.46 wt.% |
| $K_2O$ | 10.03 |
| $Cr_2O_3$ | 2.42 |
| $TiO_2$ | 0.087 |

The performance evaluation results are shown in Table 1.

Example 6

The catalysts of Example 6 was prepared by exactly the same treating procedure as that of Example 5 except that 2.0 g of titanium oxide were added during the wet mixing and kneading of the catalyst materials including iron oxide in Example 5.

The obtained catalysts had the following compositions:

Catalyst composition

| Component (wt.%) | Example 6 |
|---|---|
| $Fe_2O_3$ | 87.15 |
| $K_2O$ | 10.06 |
| $Cr_2O_3$ | 2.45 |
| $TiO_2$ | 0.34 |

The performance evaluation results are shown in Table 1, and the measuresment results of the performance change with time on stream for Example 6 are shown in Table 2.

Comparative example 3

The catalyst of Comparative example 3 was prepared by exactly the same treating procedure as that of Example 5 except that no titanium oxide was added during the wet mixing and kneading of the catalyst materials including iron oxide in Example 5.

The obtained catalyst had the following composition:

Catalyst composition

| | |
|---|---|
| $Fe_2O_3$ | 87.53 wt.% |
| $K_2O$ | 9.98 |
| $Cr_2O_3$ | 2.49 |

The performance evaluation results are shown in Table 1.

Comparative example 4

The catalyst of Comparative example 4 was prepared by exactly the same treating procedure as that of Example 5 except that 13.0 g of titanium oxide were added during the wet mixing and kneading of the catalyst materials including iron oxide in Example 6.

The obtained catalyst has the following composition:

Catalyst composition

| | |
|---|---|
| $Fe_2O_3$ | 85.58 wt.% |
| $K_2O$ | 9.74 |
| $Cr_2O_3$ | 2.43 |
| $TiO_2$ | 2.25 |

The performance evaluation results are shown in Table 1, and the measurement results of the performance change with time on stream are shown in Table 2.

Example 7 (not according to the claims)

1741 g of ferrous sulfate and 0.75 g of titanium sulfate were weighed and introduced into a 10 l beaker, and then 6 l of pure water was added with agitation to dissolve the salts (to give a solution A). Separately, 4 l of pure water was added beforehand in a 5 l beaker, and 796.5 g of sodium carbonate was gradually added with agitation to be dissolved (to give a solution B).
Next, the solution A is gradually added to the solution B with agitation at ordinary temperature to obtain a precipitiate of basic iron carbonate (the dropping period of the solution A was 60 minutes), which was left for 1-2 hours. Washing with water and filtration were repeated, whereby impurities were sufficiently removed, followed by transfer to a porcelain plate so as to dry in a dryer at 100 to 120 °C overnight. The dried material was then introduced into an electric furnace to be calcined at 400 °C for 4 hours, whereby iron oxide containing titanium oxide was obtained.
The whole amount of the iron oxide was transferred to a kneader, and as other catalysts materials 252 g of potassium carbonate, 25 g of magnesium carbonate, 134.3 g of cerium carbonate and 21 g of molybdenum oxide were weighed and introduced into the kneader. Pure water was gradually added with agitation to give a paste, and then the mixed and kneaded material was subjected to extrusion molding to a 1/8 inch (3.2mm) size with an extruder, dried in a dryer at 100 to 120 °C overnight, and calcined in an electric furnace at 600 °C for 4 hours.
The obtained catalyst had the following composition:

Catalyst composition

| | |
|---|---|
| $Fe_2O_3$ | 67.11 wt.% |
| $K_2O$ | 23.05 |
| $Ce_2O_3$ | 5.36 |
| $MoO_3$ | 2.80 |
| MgO | 1.64 |
| $TiO_2$ | 0.034 |

The performance evaluation results are shown in Table 1.

Example 8-9

The catalysts of Examples 8 and 9 were prepared by exactly the same treating procedure as that of Example 7 except that 3.4 g or 18.5 g of titanium sulfate were added during the preparation of iron oxide containing titanium oxide in Example 9.
The obtained catalysts had the following compositions:

Catalyst composition

| Component (wt.%) | Example 8 | Example 9 |
|---|---|---|
| $Fe_2O_3$ | 67.09 | 66.57 |
| $K_2O$ | 23.03 | 22.91 |
| $Ce_2O_3$ | 5.29 | 5.32 |
| $MoO_3$ | 2.83 | 2.79 |
| MgO | 1.61 | 1.58 |
| $TiO_2$ | 0.15 | 0.82 |

The performance evaluation results are shown in Table 1, and the measurement results of the performance change with time on stream for Example 9 are shown in Table 2.

Comparative example 5

The catalyst of Comparative example 5 prepared by exactly the same treating procedure as that of Example 9 except that no titanium sulfate was added during the preparation of iron oxide containing titanium oxide in Example 9.

The obtained catalyst had the following composition:

Catalyst composition

| | |
|---|---|
| $Fe_2O_3$ | 67.09 wt.% |
| $K_2O$ | 23.03 |
| $Ce_2O_3$ | 5.41 |
| $MoO_3$ | 2.85 |
| MgO | 1.61 |

The performance evaluation results are shown in Table 1.

Comparative example 6

The catalyst of Comparative example 6 was prepared by exactly the same treating procedure as that of Example 7 except that 37.5 g of titanium sulfate were added during the preparation of iron oxide containing titanium oxide in Example 9.

The obtained catalyst had the following composition:

Catalyst composition

| | |
|---|---|
| $Fe_2O_3$ | 66.02 wt.% |
| $K_2O$ | 22.74 |
| $Ce_2O_3$ | 5.29 |
| $MoO_3$ | 2.75 |
| MgO | 1.57 |
| $TiO_2$ | 1.63 |

The performance evaluation results are shown in Table 1, and the measurement results of the performance change with time on stream are shown in Table 2.

Table 1

| Performance evaluation results of catalysts | | | | | | |
|---|---|---|---|---|---|---|
| Catalyst | 570°C | | 600°C | | 620°C | |
| | Conversion % | Selectivity % | Conversion % | Selectivity % | Conversion % | Selectivity % |
| Example 1 | 49.5 | 97.0 | 68.6 | 95.4 | 76.3 | 93.5 |
| 2 | 48.3 | 97.1 | 68.7 | 95.3 | 75.0 | 93.7 |
| 3 | 46.2 | 97.5 | 66.4 | 96.2 | 76.8 | 94.0 |
| 4 | 44.0 | 97.8 | 64.5 | 96.5 | 74.8 | 94.3 |
| 5 | 47.7 | 95.0 | 64.4 | 93.3 | 74.3 | 91.0 |
| 6 | 49.3 | 95.1 | 66.2 | 93.3 | 75.4 | 91.2 |
| 7 | 49.6 | 96.8 | 67.8 | 95.3 | 76.5 | 93.5 |
| 8 | 47.3 | 96.8 | 67.5 | 95.2 | 75.8 | 93.7 |

Table 1 (continued)

| Performance evaluation results of catalysts | | | | | | |
|---|---|---|---|---|---|---|
| Catalyst | 570°C | | 600°C | | 620°C | |
| | Conversion % | Selectivity % | Conversion % | Selectivity % | Conversion % | Selectivity % |
| 9 | 44.0 | 97.2 | 64.6 | 95.7 | 75.9 | 94.0 |
| Com.ex. 1 | 38.8 | 96.9 | 60.5 | 95.6 | 71.7 | 93.8 |
| 2 | 40.1 | 97.7 | 61.5 | 96.2 | 71.3 | 94.6 |
| 3 | 41.1 | 95.0 | 60.5 | 93.4 | 71.2 | 91.3 |
| 4 | 44.2 | 95.7 | 6 4.1 | 93.7 | 73.0 | 91.7 |
| 5 | 39.2 | 96.5 | 60.1 | 95.2 | 70.9 | 93.3 |
| 6 | 42.2 | 97.4 | 63.0 | 95.8 | 73.0 | 94.1 |

Table 2

| Measurement results of the performance change with time on stream (conversion, %) | | | | | | |
|---|---|---|---|---|---|---|
| Catalyst | Time on stream (Hr.) | | | | | |
| | 100 | 150 | 200 | 300 | 400 | 500 |
| Example 2 | 75.0 | 74.5 | 74.1 | 73.7 | 73.4 | 72.9 |
| 6 | 75.4 | 75.1 | 74.8 | 74.6 | 74.3 | 74.0 |
| 9 | 75.9 | 75.8 | 75.5 | 74.7 | 74.0 | 73.1 |
| Com.ex.2 | 71.3 | 70.7 | 68.9 | 67.8 | 66.5 | 65.6 |
| 4 | 73.0 | 71.7 | 71.2 | 69.9 | 68.7 | 68.0 |
| 6 | 73.0 | 71.1 | 70.3 | 69.1 | 68.0 | 67.9 |

## Claims

**1.** An alkyl aromatic hydrocarbon dehydrogenation catalyst containing iron oxide, potassium oxide and titanium oxide as essential components wherein the iron oxide content is 40.0 to 90.0 wt.-%, the potassium oxide content is 5.0 to 30.0 wt.-% and the titanium oxide content is 0.15 to 0.95 wt.-% provided that all catalyst components are calculated as oxides, and said catalyst further contains either cerium oxide, molybdenum oxide and magnesium oxide or chromium oxide as promoter components,

wherein the chromium oxide content is 1.0 to 5.0 wt.-%

**2.** The catalyst according to claim 1, wherein the cerium oxide content is 2.0 to 20.0 wt.-%, the molybdenum oxide content is 1.0 to 10.0 wt.-%, the magnesium oxide content is 1.0 to 10.0 wt.-% provided that all catalyst components are calculated as oxides.

**3.** The catalyst according to claim 2, wherein the cerium oxide content is 4.0 to 6.0 wt.-%, the molybdenum oxide content is 2.0 to 4.0 wt.-%, the magnesium oxide content is 1.5 to 4.0 wt.-% and the chromium oxide content is 2.0 to 4.0 wt.-%, provided that all catalyst components are calculated as oxides.

**4.** The catalyst according to claim 1, wherein the alkyl aromatic hydrocarbon is selected from ethylbenzene, diethylbenzene and cumene.

**5.** A method for producing the catalyst according to claim 1, wherein the catalyst components oxides and/or catalyst component oxide precursor compounds are subjected to wet mixing and kneading followed by extrusion molding, and are subsequently dried and calcined.

## Patentansprüche

1. Dehydrierkatalysator für alkylaromatische Kohlenwasserstoffe, enthaltend Eisenoxid, Kaliumoxid und Titanoxid als wesentliche Bestandteile, worin der Eisenoxidgehalt 40,0 bis 90,0 Gew.-%, der Kaliumoxidgehalt 5,0 bis 30,0 Gew.-% und der Titanoxidgehalt 0,15 bis 0,95 Gew.-% beträgt, mit der Maßgabe, dass alle Katalysatorkomponenten als Oxide berechnet sind und dass der Katalysator zusätzlich entweder Ceroxid, Molybdänoxid und Magnesiumoxid oder Chromoxid als Promotorkomponenten enthält, wobei der Chromoxidgehalt 1,0 bis 5,0 Gew.-% beträgt.

2. Katalysator nach Anspruch 1, worin der Ceroxidgehalt 2,0 bis 20,0 Gew.-%, der Molybdänoxidgehalt 1,0 bis 10,0 Gew.-% und der Magnesiumoxidgehalt 1,0 bis 10,0 Gew.-% beträgt, mit der Maßgabe, dass alle Katalysatorkomponenten als Oxide berechnet sind.

3. Katalysator nach Anspruch 2, worin der Ceroxidgehalt 4,0 bis 6,0 Gew.-%, der Molybdänoxidgehalt 2,0 bis 4,0 Gew.-%, der Magnesiumoxidgehalt 1,5 bis 4,0 Gew.-% und der Chromoxidgehalt 2,0 bis 4,0 Gew.-% beträgt, mit der Maßgabe, dass alle Katalysatorkomponenten als Oxide berechnet sind.

4. Katalysator nach Anspruch 1, worin der alkylaromatische Kohlenwasserstoff aus Benzol, Diethylbenzol und Cumol ausgewählt ist.

5. Verfahren zur Herstellung des Katalysators nach Anspruch 1, worin die Oxide der Katalysatorkomponenten oder die Oxid-Vorläufer der Katalysatorkomponenten nass miteinander vermischt und geknetet werden, worauf eine Formung durch Extrusion und anschließend eine Trocknung und Calcinierung erfolgt .

## Revendications

1. Catalyseur de déshydrogénation d'hydrocarbures alkyl-aromatiques contenant de l'oxyde de fer, de l'oxyde de potassium et de l'oxyde de titane comme constituants essentiels, la teneur en oxyde de fer étant comprise entre 40,0 et 90,0 % en poids, le teneur en oxyde de potassium étant comprise entre 5,0 et 30,0 % en poids et la teneur en oxyde de titane étant comprise entre 0,15 et 0,95 % en poids, dans la mesure où tous les constituants du catalyseur sont calculés en tant qu'oxydes, et ledit catalyseur contient en outre soit de l'oxyde de cérium, de l'oxyde de molybdène et de l'oxyde de magnésium, soit de l'oxyde de chrome en tant que constituants promoteurs, et la teneur en oxyde de chrome étant comprise entre 1,0 et 5,0 % en poids.

2. Catalyseur selon la revendication 1, dans lequel la teneur en oxyde de cérium est comprise entre 2,0 et 20,0 % en poids, la teneur en oxyde de molybdène est comprise entre 1,0 et 10,0 % en poids et la teneur en oxyde de magnésium est comprise entre 1,0 et 10,0 % en poids, lorsque tous les catalyseurs sont calculés en tant qu'oxydes.

3. Catalyseur selon la revendication 2, dans lequel la teneur en oxyde de cérium est comprise entre 4,0 et 6,0% en poids, la teneur en oxyde de molybdène est comprise entre 2,0 et 4,0% en poids, la teneur en oxyde de magnésium est comprise entre 1,5 et 4,0% en poids et la teneur en oxyde de chrome est comprise entre 2,0 et 4,0% en poids, lorsque tous les constituants du catalyseur sont calculés en tant qu'oxydes.

4. Catalyseur selon la revendication 1, dans lequel l'hydrocarbure alkyl-aromatique est choisi parmi l'ethylbenzène, le diéthylbenzène et le cumène.

5. Procédé pour fabriquer le catalyseur selon la revendication 1, selon lequel on soumet les oxydes formant constituants du catalyseur et/ou les composés précurseurs des oxydes formant constituants du catalyseur à un mélange humide et à un malaxage, suivi par un moulage par extrusion, et qu'on les fait ensuite sécher et on les calcine.